# EUROPEAN PATENT APPLICATION

(11) **EP 4 714 972 A1**
(43) Date of publication of application: **25.03.2026**
(21) Application number: 24806344.8
(22) Date of filing: 28.04.2024
(51) Int. Cl.: C07K 16/28, A61P 35/00, C12N 5/10, C12N 15/62, C07K 19/00

(54) **ANTIBODY SPECIFICALLY BINDING TO CD38, AND PREPARATION METHOD THEREFOR AND USE THEREOF**

(30) Priority: 16.05.2023 CN 202310550892
(71) Applicant: Gracell Bioscience (Shanghai) Co., Ltd., Shanghai 200233 (CN)
(72) Inventor: ZHANG, Hua, Shanghai 200233 (CN); TANG, Jiaxing, Shanghai 200233 (CN); FEI, Fan, Shanghai 200233 (CN); SHEN, Lianjun, Shanghai 200233 (CN); CAO, Wei, Shanghai 200233 (CN)
(74) Representative: AstraZeneca Intellectual Property
(86) International application number: PCT/CN2024/090396
(87) International publication number: WO 2024/234988

(57) **Abstract**

Provided is an anti-CD38 nanobody or a binding fragment thereof. The antibody has good specificity. Furthermore, the antibody and a CD38-targeting immune effector cell prepared using the antibody show good therapeutic efficacy in treating or alleviating diseases having CD38-positive expression.

## Description

### TECHNICAL FIELD

The present invention relates to the field of engineered immunotherapy, and in particular relates to an antibody that specifically binds to CD38, and a preparation method therefor and a use thereof.

### BACKGROUND ART

Acute myelogenous leukemia (AML) is a cancer of the myeloid lineage of blood cells characterized by the rapid growth of immature blood cells ("mother cells"). These immature blood cells accumulate in the bone marrow and blood and interfere with normal blood cells. AML can spread to other organs such as the liver, spleen and brain. If left untreated, AML progresses rapidly and often becomes fatal within weeks or months. AML has several subtypes, and treatments and outcomes for the subtypes may vary. Typically, AML is initially treated with chemotherapy, sometimes together with targeted therapy drugs. Subsequently, the patient may further receive stem cell transplantation, another chemotherapy, surgery, or radiation therapy. AML is most common in the elderly. Some of the elderly are not healthy enough to receive intensive chemotherapy and therefore have poor clinical outcomes. Although current therapies for AML typically can relieve the disease, almost all patients will eventually relapse. There is a need for an effective immunotherapeutic agent to treat AML.

A chimeric antigen receptor (CAR) is an artificial receptor that mimics the function of a T cell receptor. The CAR incorporates the recognition and binding specificity of an antigen to an antibody, or a ligand to a receptor, and the ability of effector T cells to kill recognized tumor cells. The CAR is formed by sequentially connecting a signal peptide, an antigen recognition region (a ligand or single-chain antibody or Fab fragment), a transmembrane region, and a series of signal transduction regions (CD28, CD3, and CD137 intracellular signal transduction domains) of T cells. After T cells are modified, the CAR expressed on the surface of the T cells first binds to the surface antigen of tumor cells through the antigen recognition region, and then transmits an activation signal into the cells through the signal transduction regions, thereby activating the killing activity of the T cells towards the tumor cells in a targeted manner. A DNA sequence expressing the CAR is cloned into a lentiviral expression vector. T cells separated from the blood of a patient are infected with the vector, such that the corresponding CAR is expressed on the surface of the T cells. Then, the modified T cells are infused into the body of the patient again. The modified T cells can targetedly kill, in the body of the patient, tumor cells that express a relevant antigen, thus achieving the effect of eliminating the tumor cells.

Camelid-derived antibodies (or single-domain antibodies) are advantageous in terms of their stable properties, good water solubility, low immunogenicity, stronger affinity, small molecular weight, and stronger tissue penetration than conventional antibodies. Single-domain antibodies can be used as a diagnostic tool, and can also be used for the delivery of CAR-T and targeted drugs.

Therefore, there is an urgent need in the art to develop a novel CAR immune cell with higher specificity and/or lower immunogenicity and also optimal affinity for the treatment of acute myeloid leukemia.

### SUMMARY OF THE INVENTION

An object of the present invention is to provide an antibody that has high affinity and high biological activity and can specifically recognize a CD38 antigen, and a use thereof.

In a first aspect of the present invention, a nanobody that specifically binds to CD38 is provided. Complementary determining regions (CDRs) of a VHH chain of the nanobody comprise the following CDR1, CDR2 and CDR3:
(a) CDR1: the sequence thereof is as shown in SEQ ID NO: 6 or 11;
(b) CDR2: the sequence thereof is as shown in SEQ ID NO: 7, 9, 12 or 14; and
(c) CDR3: the sequence thereof is as shown in SEQ ID NO: 8, 10, 13 or 15.

In another preferred embodiment, the complementary determining regions (CDRs) of the VHH chain of the nanobody are selected from a group consisting of:
(Y1) CDR1 as shown in SEQ ID NO: 6, CDR2 as shown in SEQ ID NO: 14, and CDR3 as shown in SEQ ID NO: 15;
(Y2) CDR1 as shown in SEQ ID NO: 6, CDR2 as shown in SEQ ID NO: 9, and CDR3 as shown in SEQ ID NO: 10;
(Y3) CDR1 as shown in SEQ ID NO: 6, CDR2 as shown in SEQ ID NO: 7, and CDR3 as shown in SEQ ID NO: 8;
(Y4) CDR1 as shown in SEQ ID NO: 6, CDR2 as shown in SEQ ID NO: 9, and CDR3 as shown in SEQ ID NO: 10; and
(Y5) CDR1 as shown in SEQ ID NO: 11, CDR2 as shown in SEQ ID NO: 12, and CDR3 as shown in SEQ ID NO: 13.

In another preferred embodiment, the CDR1, CDR2, and CDR3 are separated by framework regions FR1, FR2, FR3, and FR4 of the VHH chain.

In another preferred embodiment, the nanobody that specifically binds to CD38 includes a humanized antibody, a camelid-derived antibody, and a chimeric antibody.

In another preferred embodiment, the amino acid sequence of the nanobody that specifically binds to CD38 is as shown in any one of SEQ ID NOs: 1-5.

In another preferred embodiment, the amino acid sequence of the VHH chain of the nanobody is selected from a group consisting of: SEQ ID NOs: 1-5, or a combination thereof.

In another preferred embodiment, the CDRs of the VHH chain of the nanobody comprise an amino acid sequence having at least 80%, preferably at least 90%, more preferably at least 95%, or even more preferably at least 99% sequence identity to any one of SEQ ID NOs: 1-5.

In another preferred embodiment, the amino acid sequence of the CDRs of the VHH chain of the nanobody comprises one or more amino acid substitutions, preferably conservative amino acid substitutions compared with any one of SEQ ID NOs: 1-5.

In another preferred embodiment, any amino acid sequence of the aforementioned amino acid sequences further includes a derivative sequence that optionally comprises addition, deletion, modification and/or substitution of at least one (e.g., 1 to 3, preferably 1 to 2, more preferably 1) amino acid and is capable of retaining the ability of specific binding to CD38.

In another preferred embodiment, the CD38 is human or non-human mammal CD38.

In another preferred embodiment, the CD38 is human, mouse, rat, or non-human primate (such as monkey) CD38.

In a second aspect of the present invention, a multivalent antibody or multi-epitope-targeting antibody that specifically binds to CD38 is provided, the multivalent antibody or multi-epitope-targeting antibody comprising at least one antibody component that targets a CD38 epitope, the antibody component being the anti-CD38 nanobody according to the first aspect of the present invention.

In another preferred embodiment, the anti-CD38 multivalent antibody or multi-epitope-targeting antibody comprises one or more anti-CD38 nanobodies.

In another preferred embodiment, the anti-CD38 antibody includes a monomer, a dimer (bivalent antibody), a tetramer (tetravalent antibody), and/or a multimer (multivalent antibody).

In another preferred embodiment, the anti-CD38 antibody comprises one or more VHH chains having the amino acid sequence as shown in any one of SEQ ID NOs: 1-5.

In another preferred embodiment, the anti-CD38 antibody comprises two VHH chains having the amino acid sequence as shown in any one of SEQ ID NOs: 1-5.

In another preferred embodiment, the antibody is selected from: animal derived antibodies, chimeric antibodies, humanized antibodies, or combinations thereof.

In a third aspect of the present invention, a recombinant protein is provided, the recombinant protein comprising:
(i) the nanobody that specifically binds to CD38 according to the first aspect of the present invention, or the multivalent antibody or multi-epitope-targeting antibody that specifically binds to CD38 according to the second aspect of the present invention; and
(ii) an optional tag sequence that facilitates expression and/or purification.

In another preferred embodiment, the antibody is a multivalent antibody.

In another preferred embodiment, the tag sequence includes an Fc tag, an HA tag, a Flag tag, and a 6His tag.

In another preferred embodiment, the Fc tag comprises mlgG2aFc.

In another preferred embodiment, the recombinant protein specifically binds to a CD38 protein.

In a fourth aspect of the present invention, a chimeric antigen receptor (CAR) fusion protein is provided, wherein the chimeric antigen receptor (CAR) fusion protein comprises, from the N-terminus to the C-terminus:
(i) an antigen-binding domain that specifically binds to CD38, the antigen-binding domain comprising the nanobody according to the first aspect of the present invention;
(ii) a transmembrane domain;
(iii) at least one co-stimulatory domain; and
(iv) an activation domain.

In another preferred embodiment, the antigen-binding domain is monovalent or multivalent.

In another preferred embodiment, the antigen-binding domain is from the nanobody according to the first aspect of the present invention or the recombinant protein according to the third aspect of the present invention.

In another preferred embodiment, the CAR has a structure as shown in Formula la:

L-VHH-F-H-TM-C-CD3ζ (la)

where,
each "-" is independently a linker peptide or peptide bond;
L is a signal peptide sequence;
VHH is the antigen-binding domain that specifically binds to CD38;
F is a Flag tag;
H is a hinge region;
TM is a transmembrane domain;
C is a co-stimulatory signal domain; and
CD3ζ is a cytoplasmic signal transduction sequence (including wild-type, or a mutant/modification thereof) derived from CD3ζ.

In another preferred embodiment, the L is a signal peptide derived from CD8.

In another preferred embodiment, the L comprises an amino acid sequence as shown in SEQ ID NO: 20.

In another preferred embodiment, the amino acid sequence of the VHH is as shown in any one of SEQ ID NOs: 1-5.

In another preferred embodiment, the H comprises a hinge region of CD28.

In another preferred embodiment, the TM includes a transmembrane region derived from CD28.

In another preferred embodiment, the amino acid sequence of the H and the TM comprises an amino acid sequence as shown in SEQ ID NO: 22.

In another preferred embodiment, the C is a transmembrane region of a protein selected from a group consisting of: CD28, 4-1BB, CD8a, or a combination thereof.

In another preferred embodiment, the C includes a co-stimulatory signal molecule derived from 4-1BB.

In another preferred embodiment, the C comprises an amino acid sequence as shown in SEQ ID NO: 23.

In another preferred embodiment, the CD3ζ comprises an amino acid sequence as shown in SEQ ID NO: 24.

In another preferred embodiment, the CAR fusion protein has an amino acid sequence as shown in any one of SEQ ID NOs: 25-29.

In a fifth aspect of the present invention, an antibody-drug conjugate is provided, the antibody-drug conjugate comprising:
(a) the nanobody according to the first aspect of the present invention, the multivalent antibody or multi-epitope-targeting antibody according to the second aspect of the present invention, or the recombinant protein according to the third aspect of the present invention; and
(b) a conjugated moiety that is conjugated to the antibody moiety, the conjugated moiety being selected from a group consisting of: detectable labels, drugs, toxins, cytokines, radionuclides, enzymes, or combination thereof.

In another preferred embodiment, the antibody moiety is conjugated to the conjugated moiety via a chemical bond or a linker.

In a sixth aspect of the present invention, a polynucleotide is provided, the polynucleotide encoding a protein selected from a group consisting of: the nanobody according to the first aspect of the present invention, the multivalent antibody or multi-epitope-targeting antibody according to the second aspect of the present invention, the recombinant protein according to the third aspect of the present invention, or the CAR fusion protein according to the fourth aspect of the present invention.

In a seventh aspect of the present invention, an expression vector is provided, the expression vector comprising the polynucleotide according to the sixth aspect of the present invention.

In another preferred embodiment, the expression vector is selected from a group consisting of: DNA, RNA, viral vectors, plasmids, transposons, other gene transfer systems, or combinations thereof.

In another preferred embodiment, the expression vector is a lentiviral vector.

In an eighth aspect of the present invention, a host cell is provided, the host cell comprising the expression vector according to the seventh aspect of the present invention, or having the polynucleotide according to the sixth aspect of the present invention integrated in a genome thereof, or expressing the nanobody according to the first aspect of the present invention, the multivalent antibody or multi-epitope-targeting antibody according to the second aspect of the present invention, the recombinant protein according to the third aspect of the present invention, or the CAR fusion protein according to the fourth aspect of the present invention.

In another preferred embodiment, the cell is an isolated cell, and/or the cell is a genetically engineered cell.

In another preferred embodiment, the cell is a mammalian cell.

In another preferred embodiment, the cell is a T cell.

In another preferred embodiment, the host cell is an engineered immune cell.

In another preferred embodiment, the engineered immune cell is selected from a group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells); and
(v) chimeric antigen receptor macrophages.

In a ninth aspect of the present invention, a method for preparing an engineered immune cell is provided, comprising the steps of: introducing the nucleic acid molecule according to the sixth aspect of the present invention or the vector according to the seventh aspect of the present invention into a T cell or an NK cell, so as to obtain the engineered immune cell, the engineered immune cell expressing the CAR fusion protein according to the fourth aspect of the present invention.

In another preferred embodiment, the method further comprises the step of: determining the function and efficacy of the obtained engineered immune cell.

In a tenth aspect of the present invention, a formulation is provided, the formulation comprising the nanobody according to the first aspect of the present invention, the multivalent antibody or multi-epitope-targeting antibody according to the second aspect of the present invention, the recombinant protein according to the third aspect of the present invention, the CAR fusion protein according to the fourth aspect of the present invention, the vector according to the seventh aspect of the present invention, or the host cell according to the eighth aspect of the present invention, and a pharmaceutically acceptable carrier, diluent or excipient.

In another preferred embodiment, the host cell is an engineered immune cell.

In an eleventh aspect of the present invention, a pharmaceutical composition is provided, the pharmaceutical composition comprising:
(i) the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention; and
(ii) a pharmaceutically acceptable carrier.

In another preferred embodiment, the conjugated moiety of the immunoconjugate is a drug, a toxin, and/or a therapeutic isotope.

In another preferred embodiment, the pharmaceutical composition further comprises other drugs for treating immune system diseases or tumor diseases.

In another preferred embodiment, the pharmaceutical composition is used to prepare a drug for the prevention and/or treatment of a disease or condition related to CD38.

In another preferred embodiment, the disease or condition associated with CD38 is selected from a group consisting of: acute myeloid leukemia (AML), chronic myeloid leukemia (CML), myelodysplastic syndrome (MDS), or a combination thereof.

In a twelfth aspect of the present invention, a kit is provided, the kit comprising the nucleic acid molecule according to the sixth aspect of the present invention, the recombinant protein according to the third aspect of the present invention, or the vector according to the seventh aspect of the present invention.

In another preferred embodiment, the kit is used to prepare an immune cell that expresses the receptor CAR fusion protein according to the fourth aspect of the present invention.

In a thirteenth aspect of the present invention, provided is a use of the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention in the preparation of a drug for the prevention and/or treatment of a tumor related to CD38.

In a fourteenth aspect of the present invention, a method for treating a disease is provided, comprising administering to a subject in need of treatment: the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention; and

In another preferred embodiment, the disease is a tumor having CD38-positive expression.

It should be understood that within the scope of the present invention, the above-described technical features of the present invention and the technical features specifically described below (as in Examples) can be combined with each other to form new or preferred technical solutions. Due to space limitation, such technical solutions will not be elaborated herein.

It should be understood that within the scope of the present invention, the above-described technical features of the present invention and the technical features specifically described below (as in Examples) can be combined with each other to form new or preferred technical solutions. Due to space limitation, such technical solutions will not be elaborated herein.

### BRIEF DESCRIPTION OF THE DRAWINGS

Fig. 1 shows the expression of antigens on the surface of target cells;
Fig. 2 shows the CAR positive rate of CD38-CAR T cells;
Fig. 3 shows the expression of CD38 on the surface of WT CD38-CAR T cells;
Fig. 4 shows the change in the CAR positive rate of CD38-CAR T cells during expansion culture;
Fig. 5 shows the viability and expansion factors of CD38-CAR T cells during expansion culture;
Fig. 6 shows the analysis of the ability of CD38 CAR to bind to CD38 antigen;
Fig. 7 shows the results of killing of Hela-WT and CD38-overexpressing Hela cells by CD38-CAT T (RTCA method);
Fig. 8 shows the results of killing of CD38-positive target cells by CD38-CAR T (luciferase method);
Fig. 9 shows the inhibitory effect of CD38-CAR T on Molm13 cells inoculated in immunodeficient mice and the change in the body weight of the mice after reinfusion;
Fig. 10 shows the CAR positive rate after CAR-NK92 sorting;
Fig. 11 shows the results of killing of tumor cells by CAR-NK92 (luciferase method); and
Fig. 12 shows the results of multiple rounds of killing by CAR-N92K.

### DETAILED DESCRIPTION OF PREFERRED EMBODIMENTS

After extensive and in-depth research, the present inventors unexpectedly obtained for the first time an anti-CD38 antibody having excellent affinity and high anti-tumor activity. Specifically, after extensive screening, the present inventors obtained for the first time multiple nanobodies specifically binding to CD38. On the basis of the nanobodies, recombinant antibodies and humanized antibodies, as well as CD38-targeting chimeric antigen receptors, were further prepared in the present invention. Based on an alpaca heavy chain antibody that specifically binds to CD38, the present invention provides CAR-Ts having better affinity, stronger specificity for target antigens, and lower immunogenicity compared to conventional antibodies. The CAR-T cells of the present invention have excellent target cell-killing activity and in vivo tumor-suppressing effects. On that basis, the present invention has been completed.

Specifically, in the present invention, a human-derived CD38 antigen protein is used to immunize an alpaca to obtain a high-quality immune nanobody gene library. Then, CD38 protein molecules are coupled to an ELISA plate to display the correct spatial structure of the CD38 protein. Then, using this form of antigen, the immune nanobody gene library (an alpaca heavy chain antibody phage display gene library) is screened by phage display technology, thereby obtaining a specific nanobody gene against the CD38 protein. Further, this gene is transferred into E. coli, thereby obtaining a nanobody strain that can be efficiently expressed in E. coli and has high specificity.

### Terms

In order to more easily understand the present disclosure, specific terms are first defined. As used in the present application, unless otherwise expressly indicated herein, each of the following terms shall have the meaning given below. Additional definitions are set forth throughout the application.

The term "about" may refer to a value or composition that is within an acceptable error range for a particular value or composition as determined by one of ordinary skill in the art, which will depend in part on how the value or composition is measured or determined. For example, as used herein, the expression "about 100" includes 99 and 101 and all values therebetween (such as 99.1, 99.2, 99.3, 99.4, etc.).

As used herein, the term "contain" or "include (comprise)" may be openended, semi closed-ended, and close-ended. In other words, the term also includes "substantially composed of" or "composed of".

Sequence identity is determined by comparing two aligned sequences along a predetermined comparison window (the window can be 50%, 60%, 70%, 80%, 90%, 95%, or 100% of the length of a reference nucleotide sequence or protein), and determining the number of positions where the same residue appears. Typically, sequence identity is expressed as a percentage. Methods for measuring sequence identity of nucleotide sequences is well-known for those skilled in the art.

As used herein, the terms "heavy chain variable region" and "V_{H}" can be used interchangeably.

As used herein, the terms "variable region" and "complementary determining region" (CDR) can be used interchangeably.

In the present invention, the term "antibody of the present invention", "single-domain antibody of the present invention", "protein of the present invention" or "polypeptide of the present invention" can be used interchangeably, and all refer to an antibody that specifically binds to CD38, such as a protein or polypeptide having a heavy chain variable region (such as an amino acid sequence set forth in SEQ ID NOs: 1-5). They may or may not contain a starting methionine.

### CD38

Human CD38 antigen (also called cyclic ADP ribose hydrolase) is a single-chain type II transmembrane glycoprotein with a size of 45 kDa. The overall structure can be divided into an intracellular region at the N-terminus, a single-pass transmembrane structure, and an extracellular region at the C-terminus. Its extracellular region can catalyze the synthesis and degradation of cyclic ADP-ribose (cADPR). CD38 is a marker of cell activation, and is associated with HIV infection, leukemia, myeloma, solid tumors, etc.

CD38 is detectable on the surface of many immune cells, including B cells and NK cells. CD38 is also highly expressed on the surface of a variety of tumors, including multiple myeloma, diffuse large B-cell lymphoma (DLBCL), chronic lymphocytic leukemia (CLL), acute lymphocytic leukemia (ALL), plasmacytic leukemia (PCL), acute myelogenous leukemia (AML), follicular lymphoma (FL), mantle cell lymphoma (MCL), and lung cancer, among others.

### Antibody

As used herein, the term "antibody" refers to an immunoglobulin, which is a tetrapeptide-chain structure formed of two identical heavy chains and two identical light chains connected by inter-chain disulfide bonds.

As used herein, the terms "single-domain antibody (VHH)" and "nanobody" have the same meaning, and refer to a variable region of a heavy chain of a cloned antibody. A single-domain antibody (VHH) consisting of only one heavy chain variable region is constructed, and it is the smallest antigen-binding fragment having full functions. Generally, after an antibody naturally lacking in the light chain and the heavy chain constant region 1 (CH1) is first obtained, the variable region of the heavy chain of the antibody is cloned to construct a single-domain antibody (VHH) consisting of only one heavy chain variable region.

Existing antibody numbering schemes include:
1. The Kabat scheme (Kabat et al., 1991) is based on the location of regions of high sequence variation between sequences of the same domain type. Antibody heavy (VH) and light (Vλ and Vκ) variable domains are numbered differently.
2. Chothia's scheme (Al-Lazikani, 1997) is the same as Kabat's but corrects where an insertion is annotated around the first VH complementarity determining region (CDR) so that it corresponds to a structural loop. Similarly, the Enhanced Chothia scheme (Abhinandan and Martin, 2008) makes further structural corrections of indel positions.
3. In contrast to these Kabat-like schemes, IMGT (Lefranc, 2003) and AHo (Honegger and Plückthun, 2001) both define unique schemes for antibody and T cell receptor (TCR) (Vα and Vβ) variable domains. Thus, equivalent residue positions can easily be compared between domain types. IMGT and AHo differ in the number of positions they annotate (128 and 149 respectively) and where they consider indels to occur.

Immunoglobulins differ in the composition and arrangement order of the amino acids of the heavy chain constant regions, so their antigenicity is also different. Accordingly, immunoglobulins may be divided into five classes, or immunoglobulin isotypes, namely IgM, IgD, IgG, IgA, and IgE, their corresponding heavy chains being a µ chain, a δ chain, a γ chain, an α chain, and an ε chain, respectively. Each class of Ig may be subdivided into different subclasses according to the differences in the amino acid composition of the hinge region and the number and position of the heavy chain disulfide bond. For example, IgG may be divided into IgG1, IgG2, IgG3, and IgG4. Light chains may be a κ chain or a λ chain depending on the constant region thereof. Each of the five classes of Ig may have a κ chain or a λ chain. The subunit structures and three-dimensional configurations of different classes of immunoglobulin are well-known to those skilled in the art.

The light chains of the antibody according to the present invention may further comprise a light chain constant region, the light chain constant region comprising a human or murine κ or λ chain, or a variant thereof.

In the present invention, the heavy chains of the antibody according to the present invention may further comprise heavy chain constant regions, the heavy chain constant regions comprising human or murine IgG1, IgG2, IgG3, IgG4 or variants thereof. The sequences of about 110 amino acids towards the N-terminus of the heavy chains and the light chains of the antibody vary greatly, and constitute variable regions (Fv regions); the sequences of the other amino acids towards the C-terminus are relatively stable, and constitute constant regions. The variable regions include three hypervariable regions (HVRs) and four framework regions (FRs) having a relatively conserved sequence. The three hypervariable regions determine the specificity of the antibody, and are also called complementarity determining regions (CDRs). Each light chain variable region (LCVR) and each heavy chain variable region (HCVR) consist of three CDRs and four FR regions, which are arranged in the order of FR1, CDR1, FR2, CDR2, FR3, CDR3 and FR4 from the amino terminus to the carboxyl terminus. The three CDRs of the light chains are designated as LCDR1, LCDR2 and LCDR3; the three CDRs of the heavy chains are designated as LHCDR1, HCDR2 and HCDR3.

The term "murine antibody" in the present invention refers to an anti-CD38 monoclonal antibody prepared according to knowledge and skills in the art. During preparation, a test subject is injected with the CD38 antigen, and a hybridoma expressing the antibody having the desired sequence or functional characteristics is isolated. In a preferred embodiment of the present invention, the murine CD38 antibody or an antigen-binding fragment thereof may further comprise the light chain constant region of a murine κ or λ chain, or a variant thereof, or may further comprise the heavy chain constant region of murine IgG1, IgG2, IgG3, or a variant thereof.

The term "chimeric antibody" refers to an antibody formed by fusing variable regions of a murine antibody with constant regions of a human antibody. The chimeric antibody can alleviate an immune response induced by the murine antibody.

The term "humanized antibody", also known as CDR-grafted antibody, refers to an antibody produced by grafting murine CDR sequences into human antibody variable region frameworks, that is, different types of human germline antibody framework sequences. The humanized antibody can overcome a heterogeneous reaction induced by a chimeric antibody which carries a large amount of murine protein components. Such framework sequences can be obtained from public DNA databases or published references that include germline antibody gene sequences. In order to avoid the decrease in activity resulting from the decrease of immunogenicity, the human antibody variable region framework sequences can be subjected to minimal reverse mutations or back mutations to maintain the activity.

The term "antigen-binding fragment of an antibody" (or abbreviated "antibody fragment") refers to one or more fragments of an antibody that retain the ability to specifically bind to an antigen (e.g., CD38). It has been shown that a fragment of a full-length antibody can be used to perform the antigen-binding function of the antibody. Examples of binding fragments included in the term "antigen-binding fragment of an antibody" include:
(i) Fab fragment, a monovalent fragment consisting of VL, VH, CL, and CH1 domains;
(ii) F(ab')₂ fragment, a divalent fragment comprising two Fab fragments connected by a disulfide bridge on the hinge region;
(iii) Fd fragment, consisting of VH and CH1 domains; and
(iv) Fv fragment, consisting of the VH and VL domains of a single arm of an antibody.

An Fv antibody comprises a heavy chain variable region and a light chain variable region, but does not comprise a constant region. The Fv antibody is the smallest antibody fragment having all antigen-binding sites. Generally, an Fv antibody further comprises a polypeptide linker between the VH and VL domain, and can form the structure required for antigen binding.

The term "CDR" refers to one of the six hypervariable regions, within the variable domains of an antibody, that mainly contribute to antigen binding. One of the most commonly used definitions of the six CDRs is provided by Kabat E.A et al., (1991) Sequences of proteins of immunological interest. NIH Publication, No. 91-3242).

The term "epitope" or "antigenic determinant" refers to a site on an antigen where an immunoglobulin or antibody specifically binds (e.g., a particular site on the CD38 molecule). An epitope typically comprises at least 3, 4, 5, 6, 7, 8, 9, 10, 11, 12, 13, 14, or 15 consecutive or non-consecutive amino acids in a unique spatial conformation.

The terms "specific binding", "selective binding", "selectively bind", and "specifically bind" refer to binding of an antibody to a predetermined epitope on an antigen. Typically, an antibody binds with an affinity (KD) of approximately less than 10⁻⁷ M, such as approximately less than 10⁻⁸ M, 10⁻⁹ M or 10¹⁰ M or even less.

The term "competitive binding" refers to an antibody that recognizes the same epitope (also referred to as antigenic determinant) or a portion of the same epitope on an extracellular region of CD38 and binds to the antigen as the monoclonal antibody of the present invention does. An antibody that binds to the same epitope as the monoclonal antibody of the present invention does refers to an antibody that recognizes and binds to an amino acid sequence of CD38 that is recognized by the monoclonal antibody of the present invention.

The term "KD" or "Kd" refers to the dissociation equilibrium constant of a particular antibody-antigen interaction. Typically, the antibody of the present invention binds to CD38 with a dissociation equilibrium constant (KD) of less than approximately 10⁻⁷ M, such as less than approximately 10⁻⁸ M, 10⁻⁹ M or 10⁻¹⁰ M or even less.

As used herein, the term "antigenic determinant" refers to a three-dimensional spatial site on an antigen that is discontinuous and recognized by the antibody or antigen-binding fragment of the present invention.

The present invention comprises not only the entire antibody, but also a fragment of the antibody or a fusion protein formed by the antibody with another sequence, the fragment or fusion protein having immune activity. Therefore, the present invention also includes fragments, derivatives, and analogues of the antibody.

In the present invention, the antibody includes murine, chimeric, humanized, or fully human antibodies prepared using techniques familiar to those skilled in the art. The recombinant antibodies, such as chimeric and humanized monoclonal antibodies, comprise both human and non-human portions, and can be prepared using DNA recombination techniques well-known in the art.

As used herein, the term "monoclonal antibody" refers to an antibody secreted by a clone derived from a single cell. The monoclonal antibody is highly specific and targets a single antigenic epitope. The cell may be a eukaryotic, prokaryotic or phage clonal cell line.

In the present invention, the antibody may be monospecific, bispecific, trispecific, or of greater multispecificity.

In the present invention, the antibody of the present invention further includes conserved variants thereof. The conserved variants refer to, compared with the amino acid sequence of the antibody of the present invention, polypeptides formed by replacing at most 10, preferably at most 8, more preferably at most 5, and most preferably 3 amino acids with amino acids having similar or close properties. These conservative variant polypeptides are preferably produced by performing amino acid substitutions according to Table A below.

**Table A**

| Initial residue | Representative substitution(s) | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Humanized anti-CD38 antibody

The present invention provides a humanized anti-CD38 antibody (referred to as CLDN18.2 antibody hereinafter). Specifically, the present invention provides a humanized antibody having high specificity and high affinity to CD38. The humanized antibody comprises a heavy chain and a light chain, the heavy chain comprising an amino acid sequence of a heavy chain variable region (VH), and the light chain comprising an amino acid sequence of a light chain variable region (VL).

Generally, there are two types of human framework region that could be selected for antibody humanization: one is from a known mature antibody, and the other is from a human Germline sequence. Framework regions of a known mature antibody typically comprise somatic cell mutation sites, possibly bringing about potential immunogenicity. Compared to a mature antibody, framework regions of a human Germline sequence theoretically have lower immunogenicity; moreover, the framework regions have a more flexible structure and strong plasticity, and are easy to accept different CDRs. There is a bias with respect to the usage frequency of human antibody Germline genes in the human body. An antibody humanized by selecting and using a Germline framework region having a high usage frequency has advantages such as low immunogenicity, high expression level, and stable structure.

In a preferred embodiment of the present invention, during humanization, multiple factors (including similarity, usage frequency in the human body, etc.) are considered at the same time, and after extensive experimental screening, framework regions having preferred sequences are selected to perform humanization. Human antibody Germline framework regions are selected and used to perform CDR transplantation, and the humanized antibodies thus constructed have a more stable structure, a high expression level, low immunogenicity, and higher druggability.

In another preferred embodiment, the heavy chain constant region and/or light chain constant region may be a humanized heavy chain constant region or light chain constant region. More preferably, the humanized heavy chain constant region or light chain constant region is the heavy chain constant region of human IgG1, IgG2, etc., or the human kappa or lambda light chain constant region.

In another preferred embodiment, the sequence formed by adding, deleting, modifying, and/or substituting at least one amino acid sequence is preferably an amino acid sequence having least 80%, preferably at least 85%, more preferably at least 90%, and most preferably at least 95% homology.

The antibody of the present invention may be a double-stranded or single-stranded antibody, and may preferably be a fully humanized antibody.

The antibody derivatives according to the present invention may be single-chain antibodies, and/or antibody fragments, such as Fab, Fab', (Fab')2, or other known antibody derivatives in the art, as well as any one or more of IgA, IgD, IgE, IgG, and IgM antibodies or other subtypes thereof.

The antibody of the present invention may be a humanized antibody or CDR-grafted and/or modified antibody targeting CD38.

In the above content of the present invention, the number of the amino acids added, deleted, modified and/or substituted is preferably no more than 40%, more preferably no more than 35%, more preferably 1% to 33%, more preferably 5% to 30%, more preferably 10%to 25%, and more preferably 15% to 20% of the total number of amino acids in the initial amino acid sequence.

### Preparation of antibodies

Any method suitable for producing monoclonal antibodies may be used to produce the CD38 antibody of the present invention. For example, an animal may be immunized with a joined or naturally occurring CD38 protein or a fragment thereof. Suitable immunization methods may be used, including adjuvants, immunostimulants, and repeated booster immunization, and one or more means can be used.

Any suitable form of CD38 may serve as an immunogen (antigen) for generating a non-human antibody specific to CD38, and the antibody is screened for biological activities. The immunogen may be used alone or in combination with one or more immunogenic enhancers known in the art. The immunogen may be purified from a natural source, or produced in a genetically modified cell. The DNA encoding the immunogen may either be genomic or non-genomic (such as cDNA) in terms of source. Suitable genetic vectors may be used to express the DNA encoding the immunogen. The vectors include but are not limited to adenovirus vectors, baculovirus vectors, plasmids, and non-viral vectors.

The humanized antibody may be selected from any type of immunoglobulin, including IgM, IgD, IgG, IgA, and IgE. Likewise, any type of light chain may be used in the compound and method herein. Specifically, the κ or λ chain or a variant thereof may be used in the compound and method of the present invention.

The sequence of the DNA molecule for the antibody or fragment thereof of the present invention may be obtained using conventional techniques, such as PCR amplification or genomic library screening, among other methods. In addition, the coding sequences of the light and heavy chains may be fused together to form a single-chain antibody.

Once relevant sequences are obtained, a recombination method may be used to obtain the relevant sequences in large quantities. This typically involves cloning the relevant sequences into a vector, then transferring the vector into a cell, and then isolating the relevant sequences from the proliferated host cell by a conventional method so as to obtain the relevant sequences.

In addition, the relevant sequences may also be synthesized artificially, especially when the fragments are short in length. In general, a fragment having a very long sequence may be obtained by first synthesizing a plurality of small fragments and then joining same. The DNA sequence may then be introduced into various existing DNA molecules (or such as vectors) and cells known in the art.

The term "nucleic acid molecule" refers to DNA molecules and RNA molecules. The nucleic acid molecule may be single-stranded or double-stranded, but preferably double-stranded DNA. When a nucleic acid is placed in a functional relationship with another nucleic acid sequence, the nucleic acid is "operably linked." For example, if a promoter or enhancer affects the transcription of a coding sequence, the promoter or enhancer is operably linked to the coding sequence.

The term "vector" refers to a nucleic acid molecule capable of transporting another nucleic acid to which it has been linked. In one embodiment, the vector is a "plasmid", which refers to a circular double-stranded DNA loop into which an additional DNA segment may be linked.

The present invention further relates to vectors comprising the above-mentioned suitable DNA sequence and a suitable promoter or control sequence. These vectors can be used to transform an appropriate host cell to enable the host cell to express the protein.

The term "host cell" refers to a cell into which an expression vector has been introduced. The host cell may be prokaryotic cells, such as bacterial cells; or lower eukaryotic cells, such as yeast cells; or higher eukaryotic cells, such as plant or animal cells (e.g., mammalian cells).

The step of transforming the host cell with the recombinant DNA according to the present invention may be performed using techniques well known in the art. The obtained transformant may be cultured using a conventional method, whereby the transformant expresses the polypeptide encoded by the gene of the present invention. Culture is carried out in a conventional medium under suitable conditions according to the host cell used.

Generally, the host cell obtained from the transformation is cultured under conditions suitable for the expression of the antibody of the present invention. Then, a conventional immunoglobulin purification step is performed by conventional isolation and purification means well known to a person skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of the monoclonal antibody may be determined using immunoprecipitation or an in vitro binding assay (e.g., radioimmunoassay (RIA) or enzyme-linked immunosorbent assay (ELISA)).

### Antibody formulations

An antibody has different stabilities in different formulation buffer solutions, manifested by changes in charge heterogeneity, degradation of the antibody molecule, polymerization, etc. These changes in quality properties are related to the physical and chemical properties of the antibody per se. Therefore, in the development of antibody drugs, it is necessary to screen for formulation buffer solutions that are suitable for different antibodies according to the physical and chemical properties of the antibodies. Currently, commonly used antibody formulation buffer systems include phosphate buffers, citric acid buffers, histidine buffers, etc. At the same time, different concentrations of salt ions or excipients such as sorbitol, trehalose, and sucrose as well as suitable amounts of surfactants such as Tween may be added according to the properties of the antibody so as to maintain the stability of the antibody.

### Pharmaceutical composition

The present invention further provides a composition. In a preferred embodiment, the composition is a pharmaceutical composition containing the aforementioned antibody or an active fragment, fusion protein or ADC thereof, or a corresponding CAR-T cell, and a pharmaceutically acceptable carrier. Generally, these substances may be formulated in a non-toxic, inert, and pharmaceutically acceptable aqueous carrier medium, in which the pH is typically about 5 to 8, and preferably about 6 to 8, although the pH value may vary depending on the properties of the substances formulated and the disease to be treated. The formulated pharmaceutical composition may be administered through conventional means, including (but not limited to) intratumoral, intraperitoneal, intravenous, or topical administration.

The antibody of the present invention may also be intracellularly expressed by a nucleotide sequence so as to be used for cell therapy, such as for chimeric antigen receptor T cell (CAR-T) immunotherapy, etc.

The pharmaceutical composition of the present invention may be directly used for binding to CD38 protein molecules, and thus may be used for the prevention and treatment of CD38 related diseases. In addition, other therapeutic agents may also be used at the same time.

The pharmaceutical composition of the present invention contains a safe and effective amount (such as 0.001wt% to 99wt%, preferably 0.01wt% to 90wt%, and more preferably 0.1wt% to 80wt%) of the monoclonal antibody (or a conjugate thereof) of the present invention as described above, and a pharmaceutically acceptable carrier or excipient. Such a carrier includes (but is not limited to): saline, buffer, glucose, water, glycerol, ethanol, and a combination thereof. The pharmaceutical formulation should match the mode of administration. The pharmaceutical composition of the present invention may be formulated in the form of an injection prepared, for example, by a conventional method using physiological saline or an aqueous solution containing glucose and other adjuvants. The pharmaceutical composition, such as an injection or a solution, is desirably manufactured under a sterile condition. The administration dosage of the active ingredient is a therapeutically effective amount, such as about 1 µg/kg body weight to about 5 mg/kg body weight per day. In addition, the polypeptide of the present invention may also be used in conjunction with other therapeutic agents.

When the pharmaceutical composition is used, a safe and effective amount of the pharmaceutical composition is administered to a mammal. The safe and effective amount is typically at least about 10 mg/kg body weight, and in most cases does not exceed about 50 mg/kg body weight. Preferably, the dosage is about 10 mg/kg body weight to about 20 mg/kg body weight. Of course, for specific dosages, factors such as the route of administration and the patient's health status should also be considered, all of which are within the skill of an experienced physician.

### Detection use and kit

The antibody of the present invention may be used for detection applications, such as for sample testing, so as to provide diagnostic information.

In the present invention, samples (sampled objects) used include cells, tissue samples, and biopsy specimens. The term "biopsy" used in the present invention should include all types of biopsies known to those skilled in the art. Therefore, the biopsies used in the present invention may include tissue samples prepared, for example, by endoscopic methods or by organ puncture or needle biopsy.

The samples used in the present invention include fixed or preserved cell or tissue samples.

The present invention further provides a kit containing the antibody (or a fragment thereof) of the present invention. In a preferred embodiment of the present invention, the kit further comprises a container, a user manual, a buffer, etc. In a preferred embodiment, the antibody of the present invention may be immobilized on a detection plate.

**Main advantages of the present invention include:**
(a) The single domain antibody (VHH domain) against CD38 of the present invention has high specificity and optimal affinity.
(b) The CD38 CAR T cells of the present invention exhibit high specific in vitro cytotoxicity towards CD38 positive target cells.
(c) The CD38 CAR T cells of the present invention can effectively inhibit the growth of CD38 positive tumors in vivo and exhibit a long-lasting anti-tumor effect.
(d) The CD38 CAR T cells of the present invention have knocked-out/knocked-down endogenous expression of CD38 and can effectively reduce cell fratricide and improve the in vivo and in vitro expansion of CAR-T.
(e) The antibody of the present invention can be used to specifically detect a CD38 protein.

The present invention is further described below in conjunction with specific examples. It should be understood that these examples are only used to illustrate the present invention and are not used to limit the scope of the present invention. Experimental methods without specific conditions indicated in the following examples usually follow conventional conditions, for example the conditions described in Sambrook et al., Molecular Cloning: A Laboratory Manual (New York: Cold Spring Harbor Laboratory Press, 1989), or according to conditions recommended by the manufacturers. Unless otherwise specified, percentages and parts are percentages by weight and parts by weight.

### Example 1. Screening for alpaca antibodies

Antibodies were screened from a phage antibody library. The process for establishing an alpaca antibody library is briefly described as follows: Antigen CD38 was mixed and emulsified with an adjuvant and was used to immunize an alpaca by hypodermic injection five times successively. After immunization for five times, a blood sample was taken and PBMCs were separated. Total RNA of the PBMCs was extracted and reverse transcribed into cDNA. VHH was amplified through nested PCR, and the VHH fragments were inserted into phagemids. The phagemids were amplified and harvested to obtain an antibody library.

CD38 antibodies were screened for by protein panning. The process is briefly described as follows: The CD38 antigen was coated overnight. On the next day, after blocking, the antibody library was added and incubation was performed for 1 hour. After the incubation was completed, washing was performed 8 to 10 times. Then, the bound phage was eluted and amplified, and the amplified phage was separated and purified for the next round of panning.

After enrichment occurred from the screening, host bacteria were infected with the enriched phages and plated onto a resistant plate. 96 monoclones/library were randomly picked for culture. The supernatant of the overnight culture was collected, and positive monoclones were identified by a Phage ELISA experiment. Multiple monoclones were selected for sequencing according to the OD value of the phage ELISA positive antigen well (Pro) and the difference (Pro/PVA) from the control well (PVA). Further, sequences proved to be abnormal in the sequencing were eliminated and clones having the same sequence were combined.

### Results

Five single-domain antibodies that bound to CD38 were finally obtained according to the order of affinity of the positive monoclones and the frequency of occurrence of enriched antibodies. The SEQ ID NOs corresponding to the amino acid sequences and CDRs thereof are as described in Table 1, Table 2 and Table 3 below.

**Table 1: Amino acid sequence of anti-CD38 single-domain antibodies**

| Antibody No. | Amino acid sequence (the underlined part represents CDR) | Ratio of 0D values (Pro/PVA) | SEQ ID No: |
|---|---|---|---|
| B1 | QVQLVESGGGEVQPGGSLRLSCTASGFRLDYYAIGWFRRAPGKEREGIACIDITGIH TNYGDSVMGRFTISRDDRAVYLQMNNLEAGDTAIYYCAPARFSACSALDVKLSFTSW GQGTQVTVSS | 0.65/0.18 | 1 |
| B2 | QVQLVESGGGLVQPGGSLTLSCRASGFTLDYYAIGWFRQAPGKEREAIACIDITGFH TNYGDSVSGRFTISRDNAGTTVYLQMNSLEAEDTGIYYCAPARYAACSALDVKLSFT SWGQGTQVTVSS | 1.07/0.18 | 2 |
| B3 | QVQLVESGGGLVQAGGSLRLACTASGNIFASQVMNWYRQAPGSPRELVATITPGGRT TYADSAKGRFTVATDNAKATAYLQLTSLTPEDTGVYYCHVRLFPATDYWGQGTQVTV SS | 0.15/0.05 | 3 |
| B5 | QVQLVESGGGLVQPGGSLRLSCTASGFRLDYYAIGWFRQAPGKEREAISCIDITGMH | 0.89/0.13 | 4 |
| | TNYGDSVMGRFTISRDDAKRTVYLQMNSLEAEDTGIYYCAPAQYAACSALGVKVSFT SWGQGTQVTSP | | |
| B8 | QVQLVESGGGLVQPGGSLRLSCTASGFRLDYYAIGWFRQAPGKEREAIACIDITGFH TNYGDSVSGRFTISRDNAGTTVYLQMNSLEAEDTGIYYCAPARYAACSALDVKLSFT SWGQGTQVTVSS | 1.77/0.04 | 5 |

**Table 2: Amino acid sequence of CDRs of anti-CD38 single-domain antibodies**

| Antibody No. | Full-length sequence | CDR1 | CDR2 | CDR3 |
|---|---|---|---|---|
| B1 | 1 | YYAIG | CIDITGIHTNYGDSVMG | ARFSACSALDVKLSFTS |
| B2 | 2 | YYAIG | CIDITGFHTNYGDSVSG | ARYAACSALDVKLSFTS |
| B3 | 3 | SQVMN | TITPGGRTTYADSAKG | RLFPATDY |
| B5 | 4 | YYAIG | CIDITGMHTNYGDSVMG | AQYAACSALGVKVSFTS |
| B8 | 5 | YYAIG | CIDITGFHTNYGDSVSG | ARYAACSALDVKLSFTS |

**Table 3: SEQ ID NOs of anti-CD38 single-domain antibodies**

| Antibody No. | Full-length sequence SEQ ID No: | CDR1 | CDR2 | CDR3 |
|---|---|---|---|---|
| B1 | 1 | YYAIG (SEQ ID NO:6) | CIDITGIHTNYGDSVMG (SEQ ID NO:7) | ARFSACSALDVKLSFTS (SEQ ID NO:8) |
| B2 | 2 | YYAIG (SEQ ID NO:6) | CIDITGFHTNYGDSVSG (SEQ ID NO:9) | ARYAACSALDVKLSFTS (SEQ ID NO:10) |
| B3 | 3 | SQVMN (SEQ ID NO:11) | TITPGGRTTYADSAKG (SEQ ID NO:12) | RLFPATDY (SEQ ID NO:13) |
| B5 | 4 | YYAIG (SEQ ID NO:6) | CIDITGMHTNYGDSVMG (SEQ ID NO:14) | AQYAACSALGVKVSFTS (SEQ ID NO:15) |
| B8 | 5 | YYAIG (SEQ ID NO:6) | CIDITGFHTNYGDSVSG (SEQ ID NO:9) | ARYAACSALDVKLSFTS (SEQ ID NO:10) |

### Example 2. Cell culture and construction

Molm13-Luc cells, KG1-LucG cells, HL60-LucG cells, U937-LucG cells and K562-LucG cells were all cultured using RPMI 1640 culture medium; 293T and wild-type Hela cells, Hela cells expressing CD38, Hela cells expressing CLL1, and Hela cells expressing both CLL1 and CD38 (Hela-WT, Hela-CD38, Hela-CLL1, and Hela-CLL1-CD38) were cultured using DMEM culture medium. The above culture media were both supplemented with 10% (v/v) fetal bovine serum, 100 U/ml penicillin and streptomycin, 2 mM L-glutamine, and 1 mM sodium pyruvate. The cells were cultured at 37°C and 5% CO₂ under saturated humidity.

The Hela cells expressing CD38 were a stably transformed cell line obtained by transferring CD38 antigen using a lentiviral vector, and could specifically express CD38 protein molecules; the Hela cells expressing CLL1 were a stably transformed cell line obtained by transforming CLL1 antigen using a lentiviral vector, and could specifically express CLL1 protein molecules; and the Hela cells expressing both CLL1 and CD38 were a stably transformed cell line obtained by transferring CLL1-CD38 (T2A-ligated) antigen using a lentiviral vector, and could specifically express CLL1 and CD38 protein molecules. The Molm13-Luc cells were a stably transformed cell line obtained by infecting with a lentivirus expressing firefly luciferase followed by performing screening. The KG1-LucG cells, HL60-LucG cells, U937-LucG cells and K562-LucG cells were stably transformed cell lines obtained by infecting with a lentivirus expressing firefly luciferase-GFP (T2A-ligated) followed by performing screening.

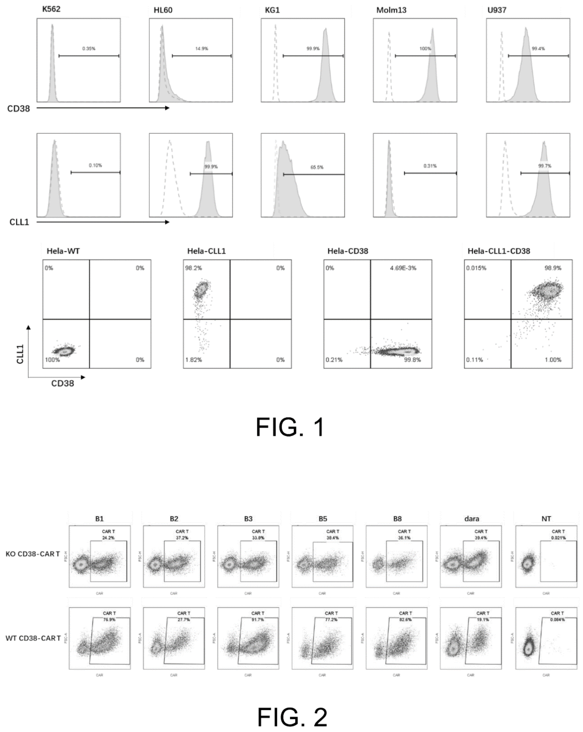

The expression of the antigens on the surface of the target cells used is as shown in Fig. 1. Hela-CD38 represents cells overexpressing CD38, Hela-CLL1 represents cells overexpressing CLL1, Hela-CLL1-CD38 represents cells overexpressing CLL1 and CD38, Hela-WT and K562 represent CLL1 and CD38-double negative cells, Molm13 represents CD38-single positive cells, and HL60, KG1 and U937 represent CLL1 and CD38-double positive cells.

### Example 3. CD38 CAR vector construction and virus preparation

The CD38 CAR gene consists of a CD8 signal peptide, a VHH or scFv that recognizes CD38, a CD8 hinge and transmembrane region, a 4-1BB signal region, and a CD3z signal region. The CD38 CAR gene was placed under the promoter of EF1α (EF-1α) to form a CD38 CAR expression vector. The CD38 CAR expression vectors were numbered B1, B2, B3, B5, and B8 according to the difference in the VHH sequence, respectively; the CD38 CAR expression vector constructed using the scFv of the CD38-targeting antibody drug daratumumab was named dara.

The amino acid sequences of the CD38C-CARs of this example are as shown in SEQ ID NOs: 25-29, and the amino acid sequence of the CAR based on the scFv of daratumumab is as shown in SEQ ID NO: 30.

2.5×10⁶ 293T cells were seeded in DMEM culture medium containing 10% FBS in a 150 cm² dish, and the cells were cultured overnight at 37°C and 5% CO₂ under saturated humidity prior to transfection.

The next day, a helper plasmid and the CD38 CAR expression vector were added to a centrifuge tube containing 13.8 mL of Opti MEM culture medium, and then 80 µg of PEI was added to the tube to obtain a mixture. The mixture was left to stand at room temperature for 20 minutes, and then supplemented with 12 mL of Opti MEM culture medium to obtain a transfection culture medium. For transfection, after removing the culture medium, the 293T cells were incubated with the transfection culture medium for 4 to 6 hours, and then the transfection culture medium was replaced with 20 mL of DMEM culture medium containing 2% FBS. After 72 hours, the culture medium was collected and centrifuged at 3000 g and 4°C for 15 min. The supernatant was further centrifuged at 27000 g and 4°C for 2 hours. The pellet was collected and resuspended with 400 µL of pre-cooled X-VIVO culture medium to obtain a CD38 CAR lentiviral suspension and kept at 4°C overnight. The next day, the virus suspension was aliquoted for further use.

### Example 4. Preparation of CAR-T cells

T cells were all cultured in X-VIVO culture medium supplemented with 1% human albumin, 4% serum replacement and 300 IU/mL human interleukin-2 (hIL-2). Pan T cells and CD3/CD28 Dynabeads at a ratio of 1:1 (CD3/CD28 Dynabeads:T cells) and 300 IU/mL of IL2 were incubated together to activate the cells. After 2 days, the activated cells were divided into two portions. One portion was directly subjected to viral transfection to prepare wild-type CD38-CAR T (WT CD38-CAR T), and the other portion was first subjected to electroporation to knock out the CD38 gene and then subjected to viral transfection to prepare CD38KO CD38-CAR T.

The preparation process of WT CD38-CAR T is as follows: After 2 days of activation with CD3/CD28 Dynabeads, the Dynabeads were removed using a magnetic column, and the T cells were directly transfected with the CD38 CAR virus at a cell density of 1 × 10⁶ cells/mL at 37°C. The transfected cells were further cultured for expansion. During the culture for expansion and before cryopreservation, the CAR positive rate of the T cells was determined using the CD38 antigen, and the expression of the CD38 on the cell surface was determined using an anti-CD38 antibody. Half of the culture medium was replaced every 2 to 3 days, and WT CD38-CAR T cells were harvested on day 8 after removing CD3/CD28 Dynabeads.

The preparation of CD38KO CD38-CAR T required electroporation of the T cells having been activated by CD3/CD28 Dynabeads for 2 days to knock out the CD38 gene. The specific operations was as follows: 0.25 nmol of gRNA targeting the CD38 gene and 16.5 ug of Cas9 protein were mixed uniformly and incubated at 37°C for 15 min to prepare RNP. During the incubation, 18 uL of supplement buffer was added to 82 uL of Nucleofector Solution to prepare 100 uL of lonza P3 electroporation buffer. At the same time, CD3/CD28 Dynabeads were removed using a magnetic column, and 1 × 10⁷ activated T cells were removed and centrifuged at 400 g for 5 min, and then the cell pellet was collected. After the incubation was completed, the 1 × 10⁷ T cells of the pellet were resuspended using 100 uL of lonza P3 electroporation buffer, and then added to RNP and mixed well. Then the mixture was transferred to an electroporation cup and placed in a lonza 2B electroporator, and electroporation was performed using FI-115 procedure. The electroporated cells were incubated with 300 IU/mL IL2 overnight.

On day 2 after electroporation, the T cells were transfected with the CD38 CAR virus at a cell density of 1 × 10⁶ cells/mL at 37°C. The transfected cells were further cultured for expansion. During the culture for expansion and before cryopreservation, the CAR positive rate of the T cells was determined using the CD38 antigen, and the expression of the CD38 on the cell surface was determined using an anti-CD38 antibody. Half of the culture medium was replaced every 2 to 3 days, and CD38KO CD38-CAR T cells were harvested on day 8 after removing CD3/CD28 Dynabeads.

Fig. 2 shows that expression of CD38 CAR could be determined, using the CD38 antigen, on the surface of the T-cells after viral transfection.

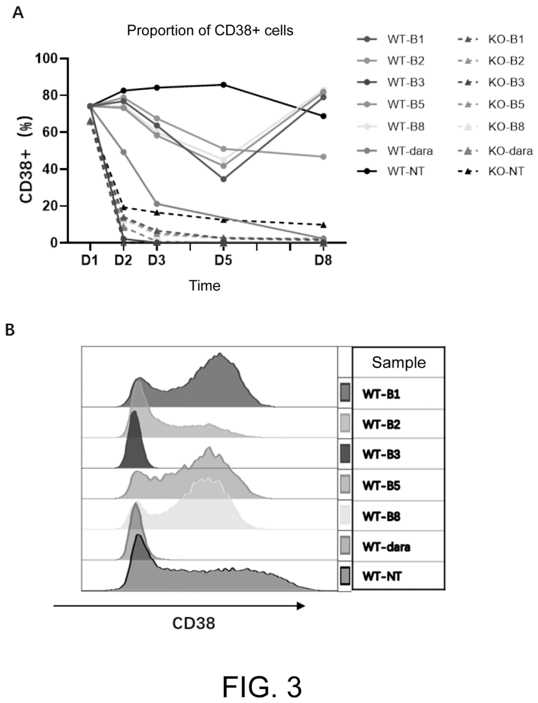

Due to the presence of the CD38 protein on the surface of the T cells, the expression of CD38 CAR would lead to self-killing, thereby leading to the elimination of the CD38-expressing T cells. Fig. 3A summarizes changes in the proportion of CD38+ cells during cell expansion culture. Among the WT CD38-CAR T cells, the proportion of CD38+ cells of B3 and dara rapidly decreased to CD38 negative, and did not recover during the culture; the proportion of CD38+ cells of B1, B2, B5, and B8 gradually decreased in the early stage of the culture, and the proportion of CD38+ cells of B1, B5, and B8 recovered in the late stage of the culture. Fig. 3B shows the expression of CD38 on the surface of WT CD38-CAR T cells in each group on the day of cryopreservation, wherein B3 and dara were CD38 negative; B1, B2, B5, and B8 were similar to NT, showing partial CD38 positivity, but the average fluorescence intensity of the CD38+ fraction of the B1, B2, B5, B8 cells was lower than that of the NT cells.

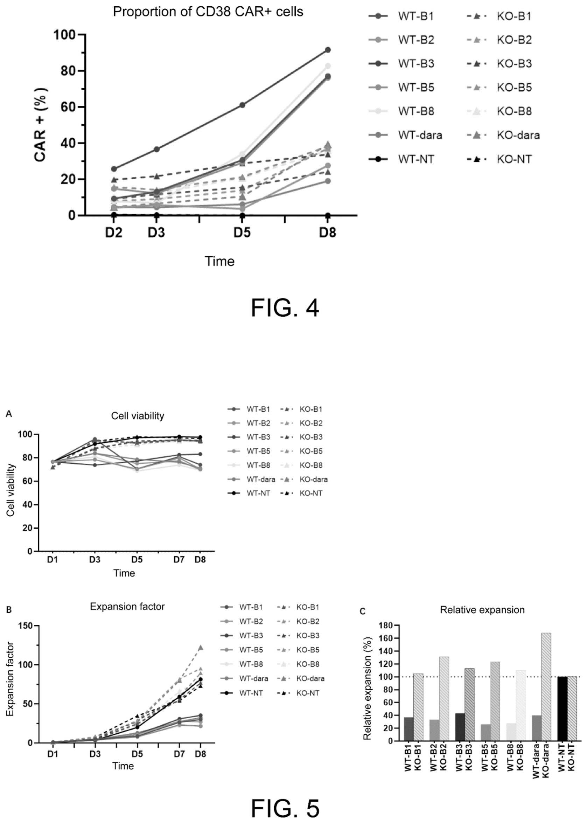

Similarly, self-killing caused by CD38 CAR expression would cause a change in the proportion of the CAR+ cells. The dynamic change in CD38 CAR expression on the cell surface is as shown in Fig. 4. In the WT CD38-CAR T cells without gene editing, the CAR positive rate was continuously up-regulated, while in the CD38KO CD38-CAR T cells, the CAR positive rate did not change drastically.

The presence of self-killing would also affect the viability and expansion of the cells. Fig. 5A summarizes the change in viability during cell expansion. It can be seen that the viability of the CD38KO CD38-CAR T cells was significantly higher than that of the WT CD38-CAR T cells. Fig. 5B analyzes the expansion factors of the cells. The expansion factor of the CD38KO CD38-CAR T cells was significantly higher than that of the WT CD38-CAR T cells. Fig. 5C analyzes the expansion of the WT (KO) CD38-CAR T cells relative to the WT (KO) NT cells. It can be seen that the expansion of the WT CD38-CAR T cells was significantly weaker than that of the WT NT cells.

### Example 5. Analysis of the ability of CD38 CAR to bind to antigen

The CD38 antigen was serially diluted, and the dilutions were used to titrate the CAR positive rate of different CD38-CAR T cells, respectively, so as to analyze the ability of different CD38 CARs to bind to the CD38 antigen.

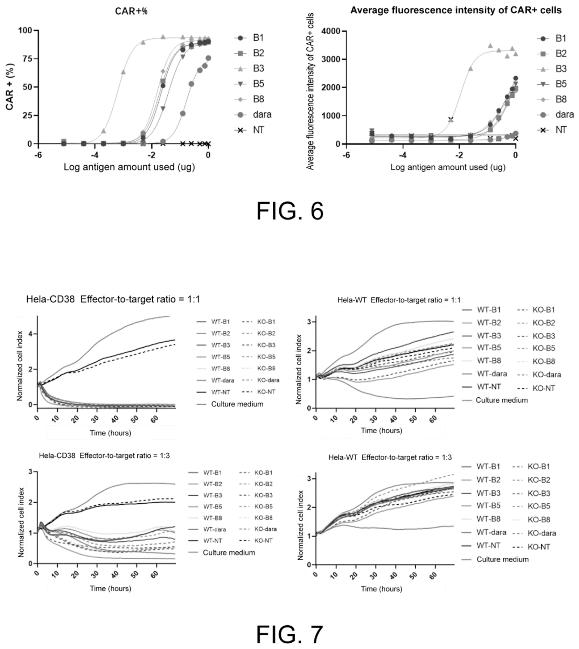

The analysis results are as shown in Fig. 6. B1, B2, B3, B5 and B8 could bind to a fewer number of the CD38 antigen than dara, and they also had higher average fluorescence intensity than dara under the condition that the amount of the antigen used was the same.

### Example 6. In vitro killing by the CD38-CAR T cells

An in vitro killing experiment was conducted on the CD38-CAR T cells obtained above. RTCA method was used to determine the killing of target cells, i.e., Hela cell line overexpressing CD38, by the CAR-T cells.

The results are as shown in Fig. 7 (determined by RTCA method). At two effector-target ratios, the NT control groups did not kill the Hela-CD38 cells, while the CD38-CAR T cells had a killing effect on Hela-CD38. In the case of the Hela-WT cells, WT-dara showed a certain degree of non-specific killing, but the other groups did not show non-specific killing against the Hela-WT cells.

Luciferase-labeled tumor target cells were used to determine the killing ability. A luciferase gene was transferred into target cells, and after cloning and screening, stably transformed cell lines KG1-LucG and K562-LucG were obtained. During the experiment, a luciferin substrate was added, and the luciferase reacted with the luciferin to produce fluorescence. The activity of the luciferase could be determined by measuring the intensity of fluorescence, and the killing effect of the respective kinds of CAR-T cells could be obtained by measuring the cell survival rate.

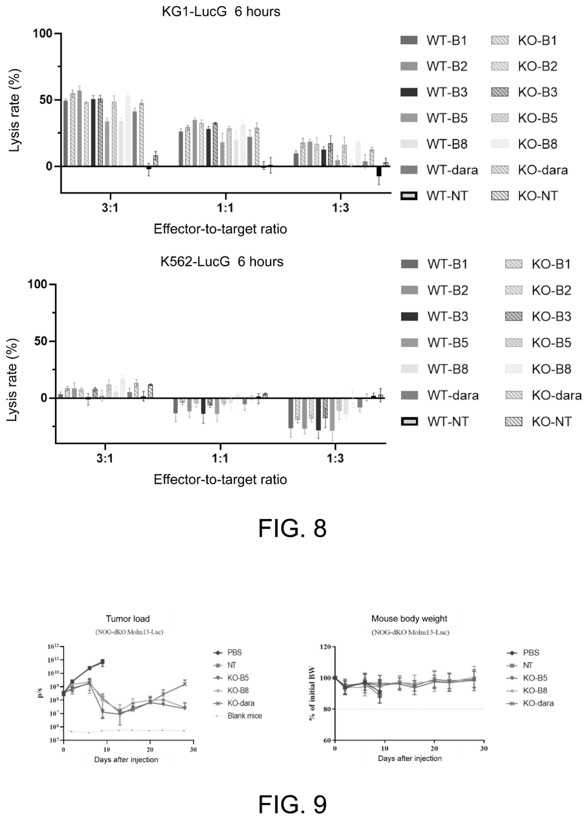

Fig. 8 shows that at all E:T ratios, the NT cells did not show the killing function, the CD38-CAR T cells had a dose-dependent killing effect on the KG1-LucG cells, and the CD38-CAR T cells did not have a killing ability against the K562-LucG cells.

In summary, after the CD38-CAR T cells were co-cultured with target cells (CD38-overexpressing Hela cells, and CD38-positive tumor cells, KG1 cells), the target cells could be lysed by the CAR T cells targeting CD38; and except for WT-dara, the other groups of CD38-CAR T cells did not show non-specific killing against non-target cells (wild-type Hela cells, and CD38-negative K562 cells).

### Example 7. Study on CD38-CAR T in vivo efficacy

Four to six week-old NOG-dKO mice were selected and injected with 2 × 10⁶ Molm13-Luc cells via tail vein. Six days later, tumor load was determined through live imaging of small animals. On the same day, the mice were grouped and injected with KO CD38-CAR T cells and NT cells. After T cells treatment, tumor load of the mice was evaluated through live imaging of small animals twice a week.

The results are as shown in Fig. 9. Compared with the NT control group, the tumor growth in the mice injected with the KO CD38-CAR T cells was inhibited. KO-B5 and KO-B8 showed a more long-lasting tumor inhibition effect than KO-dara. The reinfusion of the respective groups of CD38-CAR T cells did not have a significant effect on the body weight of the mice.

### Example 8. NK92 cells and culture method

The complete culture medium for NK92 cells was Alpha MEM supplemented with 12.5% FBS, 12.5% Horse Serum, 1% Pen/strep, 1% sodium pyruvate, 1% L-glutamine, 100U IL2.HL60, Molm13, KG-1, and THP-1, and the complete culture medium for K562 cells was RPMI1640 supplemented with 10% FBS, 1% Pen/strep, 1% sodium pyruvate, and 1% L-glutamine. The cells were cultured at 37°C and 5% CO₂ under saturated humidity.

### Example 9. Packaging and transfection of CAR-NK92 lentivirus

293T cells were resuscitated and seeded into a 150 cm² culture dish, and cultured in a CO2 incubator for 72 h. After two passages, the cells were seeded into a 150 cm² culture dish for transfection. A four-plasmid system composed of a lentiviral expression vector, helper plasmid gag/pol, Rev, and VSV-G was mixed with PEI transfection reagent, and then added to a certain volume of serum-free DMEM. The mixture was mixed uniformly and left to stand for 15 min. The resulting mixed solution was added to a 150 cm² culture dish on which the 293T cells were spread. The cells were gently mixed uniformly, and cultured in a 5% CO₂ cell incubator at 37°C for 6 h. After 6 h, the culture medium was replaced with fresh culture medium and the culture was continued, and after 48 h and 72 h, the lentiviral culture supernatant was collected for infection. The harvested supernatant was transferred to a centrifuge tube and centrifuged at 4000 rpm for 10 min with an acceleration of 9 and a deceleration of 9 to remove cell debris. All the centrifuged LVV supernatants were transferred to a 0.45 µm filter for clarification by filtration, and the filtrates were transferred to new centrifuge tubes. The clarified lentiviral supernatants were added to ultracentrifuge tubes and balanced on a balance. The balanced ultracentrifuge tubes were placed in hanging cups, the hanging cup were placed corresponding positions of the rotor, and then placed together in a ultracentrifuge for centrifugation. The centrifugation temperature was 4°C, the rotation speed was 100000 g, the centrifugation time was 90 min, and the acceleration and deceleration were set to the highest. After the ultracentrifugation was completed, the supernatants were discarded, and 0.5 mL of culture medium was added to each tube for resuspension at 2°C to 8°C for 2 h. The harvested virus was added to 1e6 NK92 and mixed in a 24-well plate, and cultured in a CO₂ incubator for 72 h.

### Example 10. CAR-NK92 flow cytometry detection and sorting

The cell suspension was removed, washed with DPBS three times, and centrifuged at 300 g for 5 min. The supernatant was discarded, an antibody mixed solution (anti-FLAG-APC, 1:100 in DPBS) was added. The mixture was mixed uniformly and placed at 4°C for staining for 30 min. The cells were washed with DPBS three times, and centrifuged at 300 g for 5 min. After resuspension in DPBS, the cells were used for flow cytometry detection and sorting. When performing sorting, PE-positive cells were selected and collected in a 5 ml flow cytometry tube. After the sorting was completed, the cells were centrifuged at 300 g for 5 min, and the supernatant was discarded. The cells were resuspended in a preheated culture medium and placed in a cell culture incubator at 37°C and 5% CO₂.

The sorted CAR-NK92 cells were stained with an anti-FLAG-APC antibody, and the expression of CAR was detected by flow cytometry.

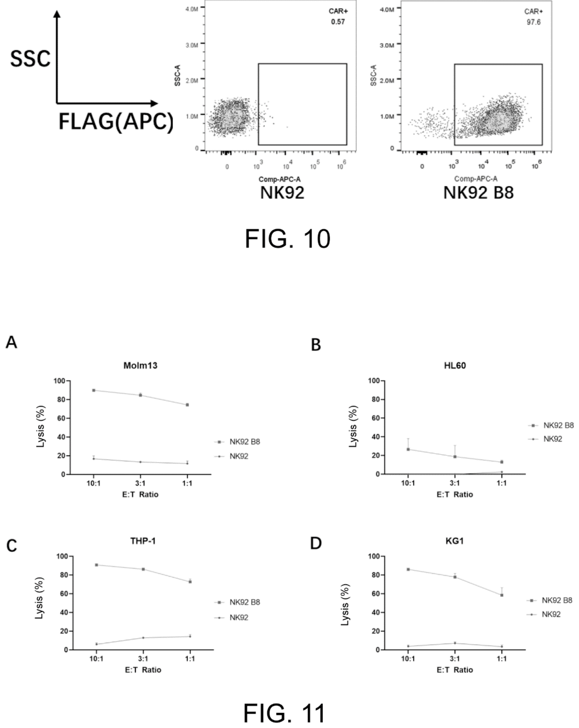

The results of the flow cytometry detection are as shown in Fig. 10. The CAR positive rate of the sorted CAR-NK92 cells reached 97% or more.

### Example 11. CAR-NK92 killing experiment

Luciferase-labeled tumor target cells were used to determine the killing ability. A luciferase gene was transferred into target cells to obtain stably transformed cell lines of Molm13, HL60, THP-1 and KG1 expressing the luciferase gene. During the experiment, a luciferin substrate was added, and luciferase reacted with the luciferin to produce fluorescence. The activity of the luciferase could be determined by measuring the intensity of fluorescence, and the killing effect of the respective kinds of CAR NK92 cells could be obtained by measuring cell survival rate.

As shown in Fig. 11, NK92 B8 showed more pronounced cytotoxicity against all target cells compared to NK92, suggesting CAR-mediated killing activity.

### Example 12. Multiple rounds of CAR-NK92 killing experiment

On the day before the experiment, the fluorescent target cells THP1 were resuspended and counted. The cell density was adjusted to 8 × 10⁴/ml, and the cells were plated on a 96-well plate at 100 ul per well. On the day of the experiment, corresponding numbers of NK92 and CAR-NK92 cells were added to the wells. The plate was placed in a Cellcyte to start the experiment. Imaging was performed every 4 hours in bright field and fluorescence channels. Every two days were taken as one round. After one round of killing was completed, part of the supernatant was removed, and the cells were transferred to a new plate on which the target cells had been spread. The plate was placed in the Cellcyte to start a new round of killing. Killing Index is calculated as the decrease in the number of fluorescent cells after making adjustments for the zero-crossing moment and for normal cell growth variation.

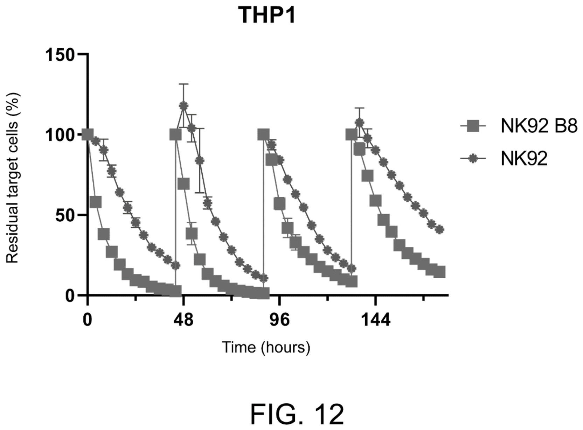

The results of the multiple rounds of killing experiment are as shown in Fig. 12. NK92 B8 cells showed a stronger killing ability than NK92 cells, suggesting that the CAR-mediated killing was continuously effective.

### Sequence information

| Description | Sequence | SEQ ID No: |
|---|---|---|
| Amino acid sequence of B1 alpaca antibody | QVQLVESGGGEVQPGGSLRLSCTASGFRLDYYAIGWFRRAPGKEREGIACIDITGIHT NYGDSVMGRFTISRDDRAVYLQMNNLEAGDTAIYYCAPARFSACSALDVKLSFTSWGQ GTQVTVSS | 1 |
| Amino acid sequence of B2 alpaca antibody | QVQLVESGGGLVQPGGSLTLSCRASGFTLDYYAIGWFRQAPGKEREAIACIDITGFHT NYGDSVSGRFTISRDNAGTTVYLQMNSLEAEDTGIYYCAPARYAACSALDVKLSFTSW GQGTQVTVSS | 2 |
| Amino acid sequence of B3 alpaca antibody | QVQLVESGGGLVQAGGSLRLACTASGNIFASQVMNWYRQAPGSPRELVATITPGGRTT YADSAKGRFTVATDNAKATAYLQLTSLTPEDTGVYYCHVRLFPATDYWGQGTQVTVSS | 3 |
| Amino acid sequence of B5 alpaca antibody | QVQLVESGGGLVQPGGSLRLSCTASGFRLDYYAIGWFRQAPGKEREAISCIDITGMHT NYGDSVMGRFTISRDDAKRTVYLQMNSLEAEDTGIYYCAPAQYAACSALGVKVSFTSW GQGTQVTVSP | 4 |
| Amino acid sequence of B8 alpaca antibody | QVQLVESGGGLVQPGGSLRLSCTASGFRLDYYAIGWFRQAPGKEREAIACIDITGFHT NYGDSVSGRFTISRDNAGTTVYLQMNSLEAEDTGIYYCAPARYAACSALDVKLSFTSW GQGTQVTVSS | 5 |
| Amino acid sequence of DarascFv light chain | | 6 |
| Amino acid sequence of DarascFv heavy chain | EVQLLESGGGLVQPGGSLRLSCAVSGFTFNSFAMSWVRQAPGKGLEWVSAISGSGGGT YYADSVKGRFTISRDNSKNTLYLQMNSLRAEDTAVYFCAKDKILWFGEPVFDYWGQGT LVTV | 7 |
| Amino acid sequence of (G4S)3 linker peptide | GGGGSGGGGSGGGGS | 8 |
| | EIVLTQSPATLSLSPGERATLSCRASQSVSSYLAWYQQKPGQAPRLLIYDASNRATGI | 9 |
| Amino acid sequence of DarascFv | PARFSGSGSGTDFTLTISSLEPEDFAVYYCQQRSNWPPTFGQGTKVEIKGGGGSGGGG SGGGGSEVQLLESGGGLVQPGGSLRLSCAVSGFTFNSFAMSWVRQAPGKGLEWVSAIS GSGGGTYYADSVKGRFTISRDNSKNTLYLQMNSLRAEDTAVYFCAKDKILWFGEPVFD YWGQGTLVTV | |
| Amino acid sequence of CD8 signal peptide | MALPVTALLLPLALLLHAARP | 10 |
| Amino acid sequence of Flag Tag | DYKDDDDK | 11 |
| Amino acid sequence of CD8 hinge transmembrane region | TTTPAPRPPTPAPTIASQPLSLRPEACRPAAGGAVHTRGLDFACDIYIWAPLAGTCGV LLLSLVITLYC | 12 |
| Amino acid sequence of 41BB costimulator y domain | KRGRKKLLYIFKQPFMRPVQTTQEEDGCSCRFPEEEEGGCEL | 13 |
| Amino acid sequence of CD3ζ | RVKFSRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | 14 |
| Amino acid sequence of B1 CAR | MALPVTALLLPLALLLHAARPQVQLVESGGGEVQPGGSLRLSCTASGFRLDYYAIGWF RRAPGKEREGIACIDITGIHTNYGDSVMGRFTISRDDRAVYLQMNNLEAGDTAIYYCA PARFSACSALDVKLSFTSWGQGTQVTVSSAAADYKDDDDKTTTPAPRPPTPAPTIASQ PLSLRPEACRPAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAYKQGQNQLYNE LNLGRREEYDVLDKRRGRDPEMGGKPRRKNPQEGLYNELQKDKMAEAYSEIGMKGERR RGKGHDGLYQGLSTATKDTYDALHMQALPPR | 15 |
| Amino acid sequence of B2 CAR | MALPVTALLLPLALLLHAARPQVQLVESGGGLVQPGGSLTLSCRASGFTLDYYAIGWF RQAPGKEREAIACIDITGFHTNYGDSVSGRFTISRDNAGTTVYLQMNSLEAEDTGIYY CAPARYAACSALDVKLSFTSWGQGTQVTVSSAAADYKDDDDKTTTPAPRPPTPAPTIA SQPLSLRPEACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPAYKQGQNQLY NELNLGRREEYDVLDKRRGRDPEMGGKPRRKNPQEGLYNELQKDKMAEAYSEIGMKGE RRRGKGHDGLYQGLSTATKDTYDALHMQALPPR | 16 |
| Amino acid sequence of B3 CAR | MALPVTALLLPLALLLHAARPQVQLVESGGGLVQAGGSLRLACTASGNIFASQVMNWY RQAPGSPRELVATITPGGRTTYADSAKGRFTVATDNAKATAYLQLTSLTPEDTGVYYC HVRLFPATDYWGQGTQVTVSSAAADYKDDDDKTTTPAPRPPTPAPTIASQPLSLRPEA CRPAAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRRGKGHDGL YQGLSTATKDTYDALHMQALPPR | 17 |
| Amino acid sequence of B5 CAR | MALPVTALLLPLALLLHAARPQVQLVESGGGLVQPGGSLRLSCTASGFRLDYYAIGWF RQAPGKEREAISCIDITGMHTNYGDSVMGRFTISRDDAKRTVYLQMNSLEAEDTGIYY CAPAQYAACSALGVKVSFTSWGQGTQVTVSPAAADYKDDDDKTTTPAPRPPTPAPTIA SQPLSLRPEACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPAYKQGQNQLY NELNLGRREEYDVLDKRRGRDPEMGGKPRRKNPQEGLYNELQKDKMAEAYSEIGMKGE RRRGKGHDGLYQGLSTATKDTYDALHMQALPPR | 18 |
| Amino acid sequence of B8 CAR | MALPVTALLLPLALLLHAARPQVQLVESGGGLVQPGGSLRLSCTASGFRLDYYAIGWF RQAPGKEREAIACIDITGFHTNYGDSVSGRFTISRDNAGTTVYLQMNSLEAEDTGIYY CAPARYAACSALDVKLSFTSWGQGTQVTVSSAAADYKDDDDKTTTPAPRPPTPAPTIA SQPLSLRPEACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPAYKQGQNQLY NELNLGRREEYDVLDKRRGRDPEMGGKPRRKNPQEGLYNELQKDKMAEAYSEIGMKGE RRRGKGHDGLYQGLSTATKDTYDALHMQALPPR | 19 |
| Amino acid sequence of Dara CAR | MALPVTALLLPLALLLHAARPEIVLTQSPATLSLSPGERATLSCRASQSVSSYLAWYQ QKPGQAPRLLIYDASNRATGIPARFSGSGSGTDFTLTISSLEPEDFAVYYCQQRSNWP PTFGQGTKVEIKGGGGSGGGGSGGGGSEVQLLESGGGLVQPGGSLRLSCAVSGFTFNS FAMSWVRQAPGKGLEWVSAISGSGGGTYYADSVKGRFTISRDNSKNTLYLQMNSLRAE DTAVYFCAKDKILWFGEPVFDYWGQGTLVTVAAADYKDDDDKTTTPAPRPPTPAPTIA SQPLSLRPEACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPAYKQGQNQLY NELNLGRREEYDVLDKRRGRDPEMGGKPRRKNPQEGLYNELQKDKMAEAYSEIGMKGE RRRGKGHDGLYQGLSTATKDTYDALHMQALPPR | 20 |

All documents mentioned herein are incorporated by reference into the present application as if each document were individually incorporated by reference. In addition, it should be understood that, after reading the above teachings of the present invention, those skilled in the art may make various changes or modifications to the present invention, and these equivalent forms also fall within the scope defined by the claims attached to the present application.

## Claims

1. A nanobody that specifically binds to CD38, **characterized in that** complementary determining regions (CDRs) of a VHH chain of the nanobody comprise the following CDR1, CDR2 and CDR3:
(a) CDR1: the sequence thereof is as shown in SEQ ID NO: 6 or 11;
(b) CDR2: the sequence thereof is as shown in SEQ ID NO: 7, 9, 12 or 14; and
(c) CDR3: the sequence thereof is as shown in SEQ ID NO: 8, 10, 13 or 15.

2. The nanobody according to claim 1, wherein the complementary determining regions (CDRs) of the VHH chain of the nanobody are selected from a group consisting of:
(Y1) CDR1 as shown in SEQ ID NO: 6, CDR2 as shown in SEQ ID NO: 14, and CDR3 as shown in SEQ ID NO: 15;
(Y2) CDR1 as shown in SEQ ID NO: 6, CDR2 as shown in SEQ ID NO: 9, and CDR3 as shown in SEQ ID NO: 10;
(Y3) CDR1 as shown in SEQ ID NO: 6, CDR2 as shown in SEQ ID NO: 7, and CDR3 as shown in SEQ ID NO: 8;
(Y4) CDR1 as shown in SEQ ID NO: 6, CDR2 as shown in SEQ ID NO: 9, and CDR3 as shown in SEQ ID NO: 10; and
(Y5) CDR1 as shown in SEQ ID NO: 11, CDR2 as shown in SEQ ID NO: 12, and CDR3 as shown in SEQ ID NO: 13.

3. The nanobody according to claim 1, wherein the amino acid sequence of the nanobody that specifically binds to CD38 is as shown in any one of SEQ ID NOs: 1-5.

4. A multivalent antibody or multi-epitope-targeting antibody that specifically binds to CD38, **characterized in that** the multivalent antibody or multi-epitope-targeting antibody comprises at least one antibody component that targets a CD38 epitope, the antibody component being the anti-CD38 nanobody according to claim 1.

5. A recombinant protein, **characterized in that** the recombinant protein comprises:
(i) the nanobody that specifically binds to CD38 according to claim 1, or the multivalent antibody or multi-epitope-targeting antibody that specifically binds to CD38 according to claim 4; and
(ii) an optional tag sequence that facilitates expression and/or purification.

6. A chimeric antigen receptor (CAR) fusion protein, **characterized in that** the chimeric antigen receptor (CAR) fusion protein comprises, from the N-terminus to the C-terminus:
(i) an antigen-binding domain that specifically binds to CD38, the antigen-binding domain comprising the nanobody according to claim 1;
(ii) a transmembrane domain;
(iii) at least one co-stimulatory domain; and
(iv) an activation domain.

7. The CAR fusion protein according to claim 6, wherein the CAR has a structure as shown in Formula la:
L-VHH-F-H-TM-C-CD3ζ (la)
where,
each "-" is independently a linker peptide or peptide bond;
L is a signal peptide sequence;
VHH is the antigen-binding domain that specifically binds to CD38; the amino acid sequence of the VHH is as shown in any one of SEQ ID NOs: 1-5;
F is a Flag tag;
H is a hinge region;
TM is a transmembrane domain;
C is a co-stimulatory signal domain; and
CD3ζ is a cytoplasmic signal transduction sequence (including wild-type, or a mutant/modification thereof) derived from CD3ζ.

8. The CAR fusion protein according to claim 6, wherein the CAR fusion protein has an amino acid sequence as shown in any one of SEQ ID NOs: 25-29.

9. An antibody-drug conjugate, **characterized in that** the antibody-drug conjugate comprises:
(a) the nanobody according to claim 1, the multivalent antibody or multi-epitope-targeting antibody according to claim 4, or the recombinant protein according to claim 5; and
(b) a conjugated moiety that is conjugated to the antibody moiety, the conjugated moiety being selected from a group consisting of: detectable labels, drugs, toxins, cytokines, radionuclides, enzymes, or combinations thereof.

10. A polynucleotide, **characterized in that** the polynucleotide encodes a protein selected from a group consisting of: the nanobody according to claim 1, the multivalent antibody or multi-epitope-targeting antibody according to claim 4, the recombinant protein according to claim 5, or the CAR fusion protein according to claim 6.

11. An expression vector, **characterized in that** the expression vector comprises the polynucleotide according to claim 10.

12. A host cell, **characterized in that** the host cell comprises the expression vector according to claim 11, or has the polynucleotide according to claim 10 integrated in a genome thereof, or expresses the nanobody according to claim 1, the multivalent antibody or multi-epitope-targeting antibody according to claim 4, the recombinant protein according to claim 5, or the CAR fusion protein according to claim 6.

13. A pharmaceutical composition, **characterized in that** the pharmaceutical composition comprises:
(i) the nanobody according to claim 1, the multivalent antibody or multi-epitope-targeting antibody according to claim 4, the recombinant protein according to claim 5, or an immune cell that expresses the CAR fusion protein according to claim 6; and
(ii) a pharmaceutically acceptable carrier.

14. A use of the nanobody according to claim 1, the multivalent antibody or multi-epitope-targeting antibody according to claim 4, the recombinant protein according to claim 5, or an immune cell that expresses the CAR fusion protein according to claim 6, **characterized in that** the use is for the preparation of a drug for the prevention and/or treatment of a disease related to CD38-positive expression.

15. A method for treating a disease, **characterized by** comprising administering to a subject in need of treatment: the nanobody according to claim 1, the multivalent antibody or multi-epitope-targeting antibody according to claim 4, the recombinant protein according to claim 5, or an immune cell that expresses the CAR fusion protein according to claim 6.
